# EUROPEAN PATENT SPECIFICATION

(11) **EP 1 332 088 B1**
(45) Date of publication and mention of the grant of the patent: **18.07.2007**
(21) Application number: 01972875.7
(22) Date of filing: 03.10.2001
(51) Int. Cl.: B65B 55/02

(54) **TROLLEY FOR AN AUTOCLAVE**
WAGEN FÜR EINEN AUTOKLAVEN
CHARIOT POUR AUTOCLAVE

(30) Priority: 04.10.2000 SE 0003564
(43) Date of publication of application: 06.08.2003
(73) Proprietor: Tetra Laval Holdings & Finance S.A., 1009 Pully (CH)
(72) Inventor: LAGERSTEDT, Jan, S-216 21 Malmö (SE)
(74) Representative: Forsberg, Lars-Ake
(86) International application number: PCT/SE2001/002141
(87) International publication number: WO 2002/028721

(56) References cited:
- US-A- 3 481 688
- US-A- 3 925 961
- US-A- 3 971 629
- US-A- 4 179 986
- US-A- 4 646 629
- US-A- 6 018 931

## Description

### Technical Field

The present invention concerns a trolley for the use in an autoclave. The trolley is designed to receive a number of packages for sterilization or retorting.

The trolley may be used for any type of product to be treated in an autoclave. However, the trolley has been especially developed to receive fibre-based cartons enclosing a food product or the like. The cartons and the content are sterilized simultaneously in the autoclave.

### Prior Art

It is previously known to place packages to be treated in an autoclave on a tray or the like.

Previously tin canes containing food products or the like has been sterilized simultaneously in an autoclave. When cartons have been used the cartons has usually been sterilized separately and the content separately and then the sterilized cartons has been filled with the sterilized food products. A new fibre-based carton has been developed, which can be sterilized in an autoclave when filled with a food product or the like. Thus, both the cartoon and the food product are sterilized simultaneously in the same way as tin cans have been sterilized previously.

### Summary of the Invention

One object of the present invention is that the trolley should not hinder that the heat is evenly distributed in the autoclave.

A further object is to have a simple loading and unloading of the packages to be retorted.

Thus, the trolley as defined by claim 1 has been developed to both make it possible to have an even heating throughout the autoclave and to have a simple loading and unloading of packages to be treated.

Further objects and advantages of the present invention will be obvious for a person skilled in the art when reading the detailed description below of preferred embodiments.

### Brief Description of the Drawings

An embodiment of the invention will be more closely described below in way of an example and by reference to the enclosed drawings, in which,
Fig. 1 is a front view of a trolley according to the invention,
Fig. 2 is a side view of the trolley of Fig. 1,

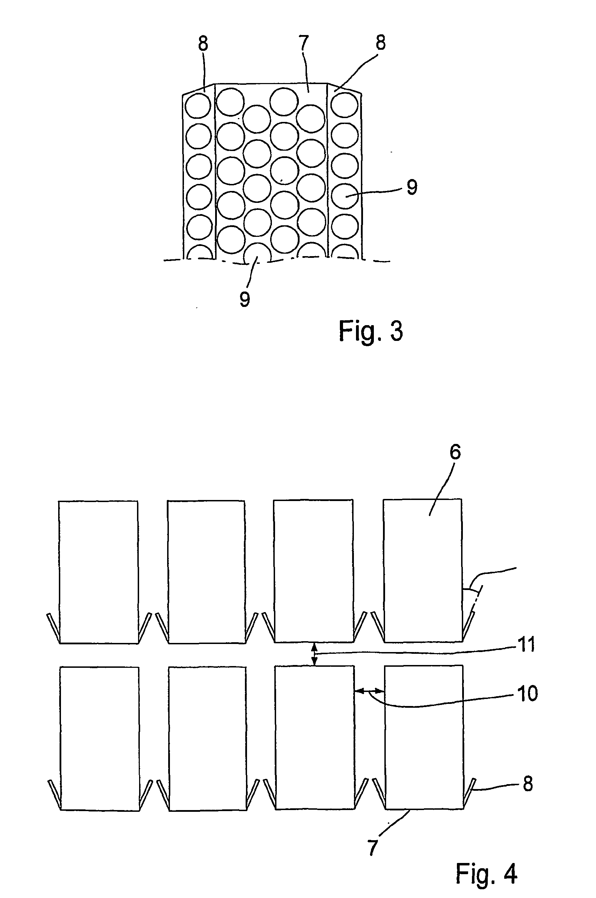

Fig. 3 is a plan view of one profile, and
Fig. 4 is a principal view showing the positions of the profiles.

### Detailed Description of Preferred Embodiments

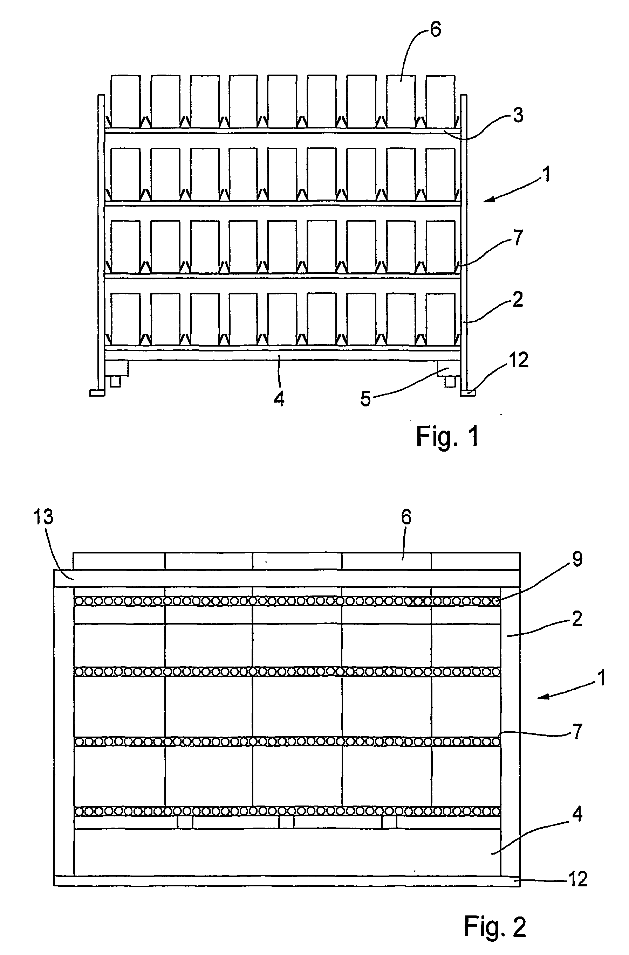

The trolley 1 of Figs. 1 and 2 has at least four vertical bars 2. The vertical bars 2 are placed at the corners of the trolley 1. On two opposite sides of the trolley 1 horizontal bars 13 go between the top of the vertical bars 2. Furthermore, a number of horizontal bars 3 are placed between the vertical bars 2 at the ends of the trolley 1. On these horizontal bars 3 a number of shelves or profiles 7 are placed. The trolley 1 has a bottom frame 4, normally furnished with four wheels 5, one in each corner. An angled bottom part 12 of the bottom frame 4 or the vertical bars 2 are adapted to support the trolley 1 outside the autoclave. Thus the trolley 1 normally rests on these bottom parts 12. In the autoclave the wheels 5 of the trolley 1 rests on rails and thus the trolley 1 is moved into and out of the autoclave by means of the wheels 5 on the rails. Outside the autoclave the trolley 1 is normally moved by means of conveyors, whereby the trolley 1 rests on the angled bottom parts 12 of the vertical bars 2 or the bottom frame 4. A person skilled in the art realizes that the trolley may have any form as long as the profiles are given the desired form and the trolley may be received by the autoclave.

The profiles 7 are to receive packages 6 that are to be treated in an autoclave and have a general U-shape. The sides 8 of the profiles 7 show an angle α in relation to a vertical plane or the side of a package 6. The profiles 7, including the sides 8, are furnished with a large number of holes 9. When designing the profiles 7 the number and size of the holes 9 is a compromise between the necessary strength of the profiles 7 and the wish that as much as possible of the packages 6 should be exposed to the steam of the autoclave.

The profiles 7 are positioned to give a distance 10 between the packages 6 in adjacent profiles 7. They also give a distance 11 in height between packages 6 placed above each other. The packages 6 in each profile 7 are normally placed abutting each other. The profiles 7 are placed parallel and with a small distance adjacent each other. The distance between the profiles 7 is normally only about 1 to 3 mm. The packages are normally placed lying down on one side to maximize the size of the surfaces of the package 6 exposed to the steam.

The holes 9 of the profiles 7 are optimised to distribute the steam and water of the autoclave in the best way. It is important that the steam and thus the heat of the autoclave is distributed evenly. Due to the holes 9 of the profiles 7 at least four sides of each package 6 are reach directly by the steam. The steam is normally distributed from the sides of the autoclave and a fan is provided to distribute the heat evenly throughout the autoclave.

The sides 8 of the profiles are angled α to facilitate loading and unloading of the packages 6 and partly to collect the cooling water. By the angled sides 8 the steam and cooling water of the autoclave will reach the packages 6 more easily. The cooling water is normally distributed from the top of the autoclave. The angle α should be 15° or more in relation to a vertical plane. Normally the angle α is between 15° and 30° and preferably between 15° and 20°. The sides 8 of the profiles 7 have a height of at least about 10 mm depending on the size of the packages 6.

The holes 9 of the profile 7 should have a diameter of about 3 to 15 mm and preferably of 4 to 10 mm. If the holes 9 have a diameter less than about 4 mm the water may be trapped in the holes 9 by means of capillary force. If the holes 9 have a diameter bigger than about 10 to 15 mm the pressure of the autoclave may deform the packages 6. The maximal allowable diameter of the holes 9 may vary depending on the packages 6, the content of the packages 6 and the temperature and pressure of the autoclave. Thus, for some packages 6 a diameter of more than 15 mm may be acceptable. A person skilled in the art realizes that the form and placement of the holes may be varied.

The size and number of profiles 7 in the trolley are adapted to the size of the packages 6 to be treated.

The packages 6 are normally pushed in a row into and out of each profile 7. The packages 6 will slide on the profile 7. The packages 6 in each profile 7 are placed abutting each other and lying on one side. A stop (not shown) is normally placed at the end of each profile and the row of packages is pushed against said stop.

In use packages 6 will be slid into each profile 7 until the desired amount of packages 6 are placed on the trolley 1. The trolley 1 is then moved into the autoclave. In the autoclave the packages 6 are first treated with steam under pressure and then cooled by water. Due to the holes 9 of the profiles 7 and the distance between the profiles 7 the steam will reach at least four sides of each package 6. After the heat treatment the packages 6 are cooled by means of water. The water is normally delivered at the top of the autoclave and will flow downwards on the trolley 1. The U-shape of the profiles 7 will collect some of the falling water and lead it towards the packages 6. The holes 9 of the profiles 7 and the distance between the profiles 7 will let the water trough and guarantee that also the lowermost packages 6 of the trolley 1 are cooled by the water.

As an alternative (not shown) the profiles 7 may be arranged on plates. Each plate is placed on top of another plate when loaded with packages 6. To keep a proper distance 11 between the packages the plates are furnished with spacers placed at the corners. The spacers may be integrated with the plates or may be lose spacers placed manually or by a machine between successive layers of plates having profiles 7 and packages 6. In this embodiment the packages 6 are normally lifted onto the desired positions in the profiles 7.

## Claims

1. A trolley (1) for receiving packages (6) to be treated in an autoclave, which is furnished with profiles (7) each adapted to receive a row of packages (6) and that at least four sides of each package (6) can be exposed to steam and cooling water of the autoclave, the cooling water being delivered at the top of the autoclave, wherein the profiles (7) U-shaped, one side of the packages can rest against the bottom of the profile, and the profiles (7) are furnished with holes (9) **characterized in that** the sides of the profiles (7) have an angle (α) of 15° to 30° in relation to a vertical plane so that the profiles being able to collect some of the falling cooling water and lead it towards the row of packages.

2. The trolley (1) of claim 1, **characterized in that** the form and positions of the profiles (7) are adapted to the form and size of the packages (6) to be treated.

3. The trolley (1) of claim 1 or 2, **characterized in that** the sides of the profiles (7) has an angle (α) of 15° to 20° in relation to a vertical plane.

4. The trolley (1) of any of the previous claims, **characterized in that** the diameter of each hole (9) is between about 3 and 15 mm and preferably between 4 and 10 mm.

5. The trolley (1) of any of the previous claims, **characterized in that** the profiles (7) are positioned parallel with each other on different heights of the trolley (1) in such a way that several profiles (7) are arranged at each height and that the positions of the profiles (7) are adapted to the size of the packages (6) to be treated in the autoclave in such a way that there is a distance (10) between packages (6) on adjacent profiles (7) on the same height and a distance (11) between packages (6) on adjacent profiles (7) on different heights.

6. The trolley (1) of claim 5, **characterized in that** the distance (10,11) between the packages (6) on adjacent profiles (7) is at least 6 mm.

7. The trolley (1) of any of the previous claims, **characterized in that** the trolley (1) has four wheels (5) for co-operation with rails in the autoclave.

8. The trolley (1) of any of the previous claims, **characterized in that** the profiles (7) are arranged on plates which are placed on top of each other.

9. The trolley (1) of claim 8, **characterized in that** spacers are placed between the plates.

10. The trolley (1) of claim 9, **characterized in that** the spacers are integrated with the plates.

## Patentansprüche

1. Wagen (1) zur Aufnahme von Verpackungen (6), die in einem Autoklaven zu behandeln sind, der mit Profilen (7) ausgestattet ist, die jeweils zur Aufnahme einer Reihe von Verpackungen (6) geeignet sind, und dass wenigstens vier Seiten jeder Verpackung (6) Dampf und Kühlwasser des Autoklavs ausgesetzt sein können, wobei das Kühlwasser am oberen Teil des Autoklaven zugeführt wird, wobei die Profile (7) U-förmig sind, eine Seite der Verpackungen am Boden des Profils anliegen kann, und die Profile (7) mit Löchern (9) ausgestattet sind, **dadurch gekennzeichnet, dass** die Seiten der Profile (7) einen Winkel (α) von 15° bis 30° bezogen auf eine vertikale Ebene haben, so dass die Profile dazu in der Lage sind, etwas von dem fallenden Kühlwasser zu sammeln und es in Richtung der Reihe aus Verpackungen zu leiten.

2. Wagen (1) nach Anspruch 1, **dadurch gekennzeichnet, dass** Form und Positionen der Profile (7) der Form und Größe der zu behandelnden Verpackungen (6) angepasst sind.

3. Wagen (1) nach Anspruch 1 oder 2, **dadurch gekennzeichnet, dass** die Seiten der Profile (7) einen Winkel (α) von 15° bis 30° bezogen auf eine vertikale Ebene haben.

4. Wagen (1) nach einem der vorhergehenden Ansprüche, **dadurch gekennzeichnet, dass** der Durchmesser jedes Lochs (9) zwischen etwa 3 und 15 mm beträgt und bevorzugt zwischen 4 und 10 mm.

5. Wagen (1) nach einem der vorhergehenden Ansprüche, **dadurch gekennzeichnet, dass** die Profile (7) parallel zueinander auf unterschiedlichen Höhen des Wagens (1) dergestalt angeordnet sind, dass mehrere Profile (7) auf jeder Höhe angeordnet sind, und dass die Positionen der Profile (7) an die Größe der in dem Autoklaven zu behandelnden Verpackungen (6) so angepasst sind, dass ein Abstand (10) zwischen Verpackungen (6) auf benachbarten Profilen (7) auf derselben Höhe besteht sowie ein Abstand (11) zwischen Verpackungen (6) auf benachbarten Profilen (7) auf unterschiedlichen Höhen.

6. Wagen (1) nach Anspruch 5, **dadurch gekennzeichnet, dass** der Abstand (10, 11) zwischen den Verpackungen (6) auf angrenzenden Profilen (7) mindestens 6 mm beträgt.

7. Wagen (1) nach einem der vorhergehenden Ansprüche, **dadurch gekennzeichnet, dass** der Wagen (1) vier Räder (5) hat, die mit den Schienen im Autoklaven zusammenwirken.

8. Wagen (1) nach einem der vorhergehenden Ansprüche, **dadurch gekennzeichnet, dass** die Profile (7) auf übereinander liegenden Platten geordnet sind.

9. Wagen (1) nach Anspruch 8, **dadurch gekennzeichnet, dass** Abstandshalter zwischen den Platten angeordnet sind.

10. Wagen (1) nach Anspruch 9, **dadurch gekennzeichnet, dass** die Abstandshalter in den Platten integriert sind.

## Revendications

1. Chariot (1) pour recevoir des emballages (6) à traiter dans un autoclave, qui est équipé de profilés (7) prévus chacun pour recevoir une rangée d'emballages (6) et de sorte qu'au moins quatre côtés de chaque emballage (6) peuvent être exposés à la vapeur et à l'eau de refroidissement de l'autoclave, l'eau de refroidissement étant distribuée à la partie supérieure de l'autoclave, dans lequel les profilés (7) sont en forme de U, un côté des emballages peut reposer contre le fond du profilé, et les profilés (7) comportent des trous (9), **caractérisé en ce que** les côtés des profilés (7) définissent un angle (α) de 15 degrés à 30 degrés par rapport à un plan vertical de sorte que les profilés peuvent collecter une partie de l'eau de refroidissement qui tombe et la diriger vers la rangée d'emballages.

2. Chariot (1) selon la revendication 1, **caractérisé en ce que** la forme et les positions des profilés (7) sont adaptées à la forme et à la dimension des emballages (6) à traiter.

3. Chariot (1) selon la revendication 1 ou 2, **caractérisé en ce que** les côtés des profilés (7) forment un angle (α) de 15 degrés à 20 degrés par rapport à un plan vertical.

4. Chariot (1) selon une quelconque des revendications précédentes, **caractérisé en ce que** le diamètre de chaque trou (9) est compris entre 3 et 15 mm environ et de préférence entre 4 et 10 mm.

5. Chariot (1) selon une quelconque des revendications précédentes, **caractérisé en ce que** les profilés (7) sont placés parallèlement les uns aux autres à différents niveaux du chariot (1) d'une manière telle que plusieurs profilés (7) sont disposés à chaque niveau et que les positions des profilés (7) sont adaptées à la dimension des emballages (6) à traiter dans l'autoclave, de sorte qu'il y a une distance (10) entre les emballages (6) placés sur des profilés adjacents (7) au même niveau, et une distance (11) entre les emballages (6) placés sur des profilés adjacents (7) à différents niveaux.

6. Chariot (1) selon une quelconque des revendications précédentes, **caractérisé en ce que** la distance (10, 11) entre les emballages (6) sur des profilés adjacents (7) est au moins de 6 mm.

7. Chariot (1) selon une quelconque des revendications précédentes, **caractérisé en ce que** le chariot (1) comporte quatre roues (5) pour coopération avec des rails dans l'autoclave.

8. Chariot (1) selon une quelconque des revendications précédentes, **caractérisé en ce que** les profilés (7) sont agencés sur des plaques qui sont placées les unes au-dessus des autres.

9. Chariot (1) selon la revendication 8, **caractérisé en ce que** des cales d'espacement sont placées entre les plaques.

10. Chariot (1) selon la revendication 9, **caractérisé en ce que** les cales d'espacement sont intégrées avec les plaques.
